# EUROPEAN PATENT APPLICATION

(11) **EP 0 756 853 A1**
(43) Date of publication of application: **05.02.1997**
(21) Application number: 96304647.9
(22) Date of filing: 24.06.1996
(51) Int. Cl.: A61F 2/06

(54) **Composite metal and polymer locking stents for drug delivery**

(30) Priority: 01.08.1995 US 510132
(71) Applicant: ADVANCED CARDIOVASCULAR SYSTEMS, INC., Santa Clara California 95052 (US)
(72) Inventor: Williams, Michael S., Chapel Hill, North Carolina 27516 (US); Campbell, Patrick K., Georgetown, Massachusetts 01833 (US)
(74) Representative: Mayes, Stuart David

(57) **Abstract**

The composite locking stent (10) includes a metal structural member (12) laminated between two layers (24) of a polymeric material capable of carrying and delivering therapeutic drugs. The metal and polymer components are incorporated into a rolled locking design. The head end (16) includes a slot (22) for receipt of the tail end (20), so that the tail end and main body portion are insertable through the slot so as to form a cylindrical loop. The tail end preferably includes a plurality of teeth (23) adapted to cooperatively engage the slot of the head end for retaining the tail end when inserted in the slot. The metal structural member can function as the mechanical structural backbone and a radio-opaque visualization marker, or with a polymeric material sufficiently strong to provide the structural strength required for a stent, the metal structural member can simply be provided to function as a radio-opaque visualization marker.

## Description

### BACKGROUND OF THE INVENTION

### Field of the Invention

This invention relates generally to expandable intraluminal vascular grafts, commonly referred to as stents and, more particularly, concerns composite locking stents having a polymeric material component capable of carrying and releasing therapeutic drugs.

### Description of Related Art

Stents typically are implanted within a vessel in a contracted state and expanded when in place in the vessel in order to maintain patency of the vessel to allow fluid flow through the vessel. Ideally, implantation of such stents is accomplished by moving the stent along a guide wire previously placed in the vessel, and expanding and locking the stent in an expanded state by inflation of a balloon within the stent. The stent then can be left in place by deflating the balloon and removing the guide wire.

Stents commonly have a metallic structure to provide the strength required to function as a stent, but typically cannot deliver localized therapeutic pharmacological agents to a blood vessel at the location being treated with the stent. While polymeric materials that can be loaded with and release drugs or other pharmacological treatments can be used for drug delivery, polymeric materials may not fulfill the structural and mechanical requirements of a stent, especially when the polymeric materials are loaded with a drug, because drug loading of a polymeric material significantly can affect the structural and mechanical properties of the polymeric material. Because it often is useful to provide localized therapeutic pharmacological treatment of a blood vessel at the location being treated with the stent, it would be desirable to form a composite locking stent containing a structural component to provide the structure and mechanical strength required for a stent, with a polymeric component to provide the capability of being loaded with therapeutic drugs, to function together as a stent for placement and release of the therapeutic drugs at a specific intravascular site.

### SUMMARY OF THE INVENTION

Preferred embodiments of the present invention provide for a composite locking stent offering structural strength and drug loading and delivery. The composite locking stent can be implanted in the vasculature for delivering therapeutic drugs at a specific site of vascular injury, such as can occur from percutaneous transluminal coronary angioplasty ("PTCA") or de novo lesions of atherosclerotic disease. The currently preferred composite locking stent incorporates a metal substructure which can provide the bulk of the strength of the stent. The metal may vary in its type, geometry and function depending on what properties are required. Preferred embodiments provide for the desired characteristics of a structurally sound drug delivery stent by forming a composite of a metal stent structure laminated between two layers of a polymeric material that is capable of carrying and delivering therapeutic drugs, and incorporating the metal and polymer composite into a rolled locking design.

One embodiment accordingly provides for a composite locking stent, comprising a proximal head end portion, and at least one elongated main body portion connected to the head end portion. Each main body portion extends distally from the head end portion, with each main body portion having a tail end portion opposing the head end portion. At least one elongated slot is disposed in the head end portion extending transversely to the elongated main body portion, and each tail end portion is adapted to interlock with the head end portion through a corresponding one of the slots in the head end portion. Metal structural means are disposed in at least a portion of one of the head end portion and the at least one main body portion, with the metal structural means being disposed between first and second layers of polymeric material laminated together. The first and second layers of polymeric material form an exterior portion of the head end portion and each main body portion, and at least one of the layers of polymeric material is capable of being loaded with and releasing therapeutic agents at a predictable rate for delivery of the therapeutic agents in localized drug therapy in a blood vessel. The metal structural means preferably comprises a planar metal structural member, and can function as the mechanical structural backbone in the head end and main body portion or alternatively in only the head end, as well as a radio-opaque visualization marker, or with a polymeric material sufficiently strong to provide the structural strength required for a stent, the metal structural member can simply be provided to function as a radio-opaque visualization marker. The tail end portion of each main body portion preferably has peripheral edges with a plurality of teeth directed outward from the tail end portion and distally from the head end portion for engaging the corresponding slot in the head end portion, and the teeth can be formed by the metal structural member or the polymeric material. The teeth are adapted to cooperatively engage the slot of the head end for retaining the tail end when inserted in the slot, so that the stent can be placed in a blood vessel in a contracted cylindrical loop shape, urged into an expanded configuration, such as by an inflation balloon, and locked in the expanded configuration by the interlocking of the teeth of tail end with the slot. The metal structural member preferably has a surface defining at least one aperture in the each main body portion, and the apertures in the metal structural member preferably are filled in by the layers of polymeric material, which in turn also preferably contain a plurality of apertures. The metal and polymer composite locking stent preferably is formed as a sheet in a shape with head and tail portions so that the tail end and main body portion are insertable through the slot so as to form a rolled, expandable, cylindrical loop, that can lock in an expanded configuration.

The two polymeric layers between which the metal structural portion is laminated preferably are bioabsorbable, and at least one of the polymeric layers can be loaded with a pharmacologic agent for use in localized drug therapy. The primary purpose of the polymeric layers is to carry one or more therapeutic drugs, and to provide a launch platform for localized delivery of the therapeutic drugs.

These and other aspects and advantages of the invention will become apparent from the following detailed description, and the accompanying drawings, which illustrate by way of example the features of the invention.

### BRIEF DESCRIPTION OF THE DRAWINGS

Fig. 1 is a top plan view of a first embodiment of a composite locking stent according to the invention;
Fig. 2 is a top plan view of the metal structural member of the composite locking stent of Fig. 1;
Fig. 3 is a top plan view of a second embodiment of a composite locking stent according to the invention;
Fig. 4 is a top plan view of the metal structural member of the composite locking stent of Fig. 3;
Fig. 5 is a top plan view of a third embodiment of a composite locking stent according to the invention;
Fig. 6 is a top plan view of the metal structural member of the composite locking stent of Fig. 5; and
Fig. 7 is a top plan view of a fourth embodiment of a composite locking stent according to the invention, incorporating a metal radio-opaque marker.

### DETAILED DESCRIPTION OF THE PREFERRED EMBODIMENTS

Stents that have a metallic structure typically do not provide for the delivery of localized therapeutic drugs in a blood vessel, while polymeric materials that can be used for drug delivery may not fulfill the structural and mechanical requirements of a stent.

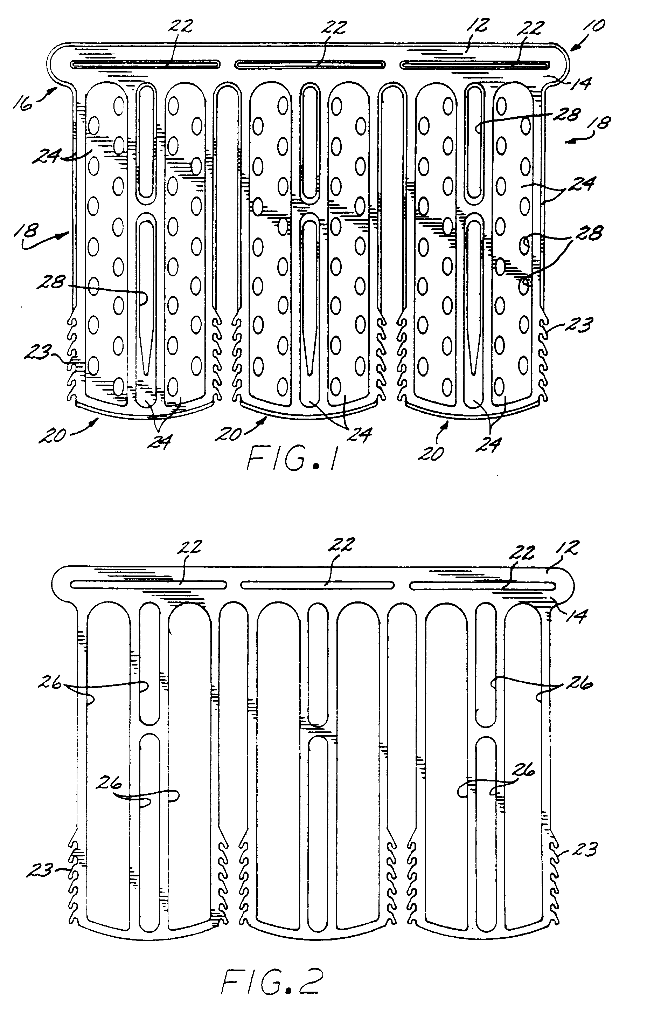

As is shown in the drawings, for purposes of illustration, preferred embodiments of the invention provide for a composite locking stent with a metal structural member that is laminated between two layers of polymeric material. At least one of the layers of polymeric material can be loaded with a pharmacologic agent or drug for delivery of the therapeutic agent or drug in localized drug therapy in the blood vessel. With reference to Figs. 1 and 2, in a first embodiment, the metal and polymer composite locking stent 10 comprises a metal structural member 12 having top and bottom surfaces 14 which essentially are a mirror image of each other, a proximal first or head end 16, and a plurality of main body portions 18 connected to the head end and extending distally from the head end. The first embodiment of the composite locking stent currently typically has three main body portions, although the composite locking stent can have as few as one main body portion, or more than three. Each of the main body portions includes a distal second end or tail end portion 20 opposing the head end.

The head end preferably includes a number of slots 22 corresponding in number and width to the main body portions of the composite locking stent. The metal structural member is formed so as to be capable of being rolled up into a cylindrical configuration whereby the first end overlaps the second end, and so that the tail end portions of the composite locking stent can be inserted through the corresponding slots in the head end. In the first embodiment, at each side of the tail end portions, the periphery of the tail end portions of the metal structural member includes a plurality of pronged teeth 23 generally projecting outward and distally from the head end. The metal structural member preferably is laminated on the exterior top and bottom surfaces with a layer of a polymer 24 capable of being loaded with and releasing therapeutic drugs. The polymeric layers of material extend beyond the peripheral edge of the metal structural member except at the tail end portions, where the teeth extend beyond the polymer material.

The layers of biodegradable polymeric film on either side of the metal structural member preferably are selected for their ability to be loaded with and deliver drugs at predictable rates when the stent is implanted in a blood vessel or other lumen in the body. The biodegradable polymeric film layers can contain the same or different drugs, or combinations of drugs, which are described further below. The polymeric materials with which the composite locking stent are laminated also will be described further below.

The main body portions of the metal structural member also preferably include a plurality of apertures 26 which can be filled in by the laminating polymer layers. The laminating polymer layers filling the metal frame apertures also preferably include apertures 28 to facilitate the process of degradation and absorption of the stent once it is implanted, to allow blood flow through the stent to side branch vessels, for blood flow to the vessel wall, for endothelialization, and flexibility.

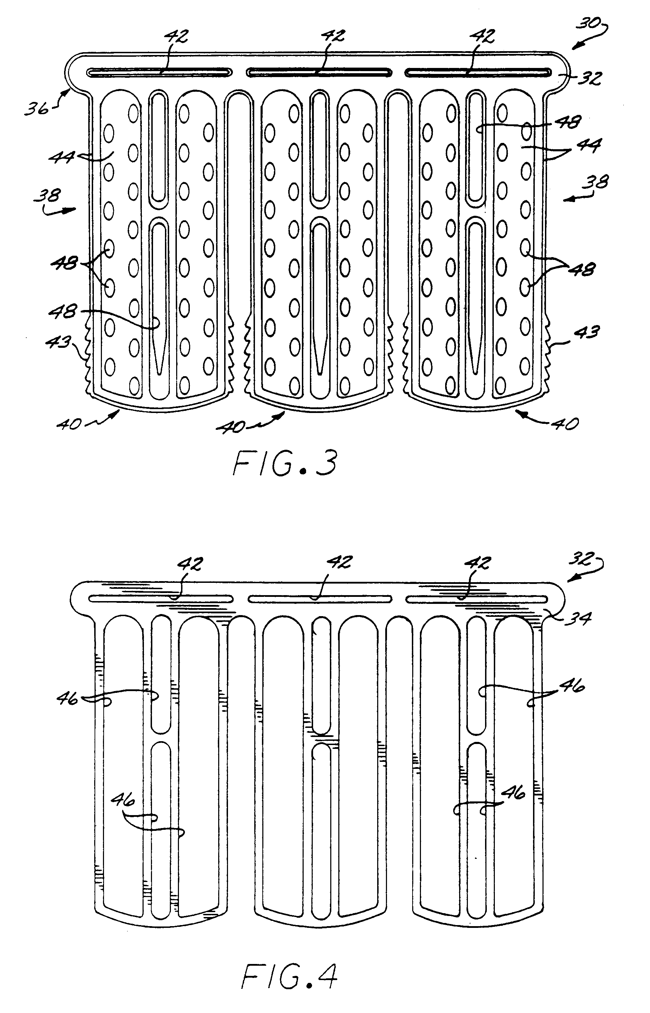

With reference to Figs. 3 and 4, in a second embodiment, the metal and polymer composite locking stent 30 comprises a metal structural member 32 having top and bottom surfaces 34 which essentially are a mirror image of each other, a proximal first or head end 36, and a plurality of main body portions 38 connected to the head end and extending distally from the head end. The second embodiment of the composite locking stent currently typically also has three main body portions, although the composite locking stent can have as few as one main body portion, or more than three. Each of the main body portions includes a distal second end or tail end portion 40 opposing the head end.

The head end preferably includes a number of slots 42 corresponding to the number and width of the main body portions of the composite locking stent. As in the first embodiment, the metal structural member is formed so as to be capable of being rolled up into a cylindrical configuration whereby the first end overlaps the second end, and so that the tail end portions of the composite locking stent can be inserted through the corresponding slots in the head end. The metal structural member preferably is laminated on the top and bottom surfaces with a layer of a polymer material 44 capable of being loaded with and releasing therapeutic drugs.

In the second embodiment, the polymeric layers extend beyond the entire peripheral edge of the metal structural member, and at each side of the tail end portions the polymeric material is formed as a plurality of scalloped or lobed teeth 43 generally directed outward and distally from the head end.

The main body portions of the metal structural member also preferably include a plurality of apertures 46 which can be filled in by the laminating polymer layers. The laminating polymer layers filling the metal frame apertures also preferably include apertures 48 to facilitate the process of degradation and absorption of the stent once it is implanted, to allow blood flow through the stent to side branch vessels, for blood flow to the vessel wall, for endothelialization, and flexibility.

The polymeric materials with which the composite locking stent are laminated in the second embodiment of the composite locking stent will be described further below. The layers of biodegradable polymeric film on either side of the metal structural member preferably are selected for the ability of the layers to be loaded with and to release drugs at predictable rates when the stent is implanted in a blood vessel or other lumen in the body. The biodegradable polymeric film layers can contain the same or different drugs, or combinations of drugs, which are described further below.

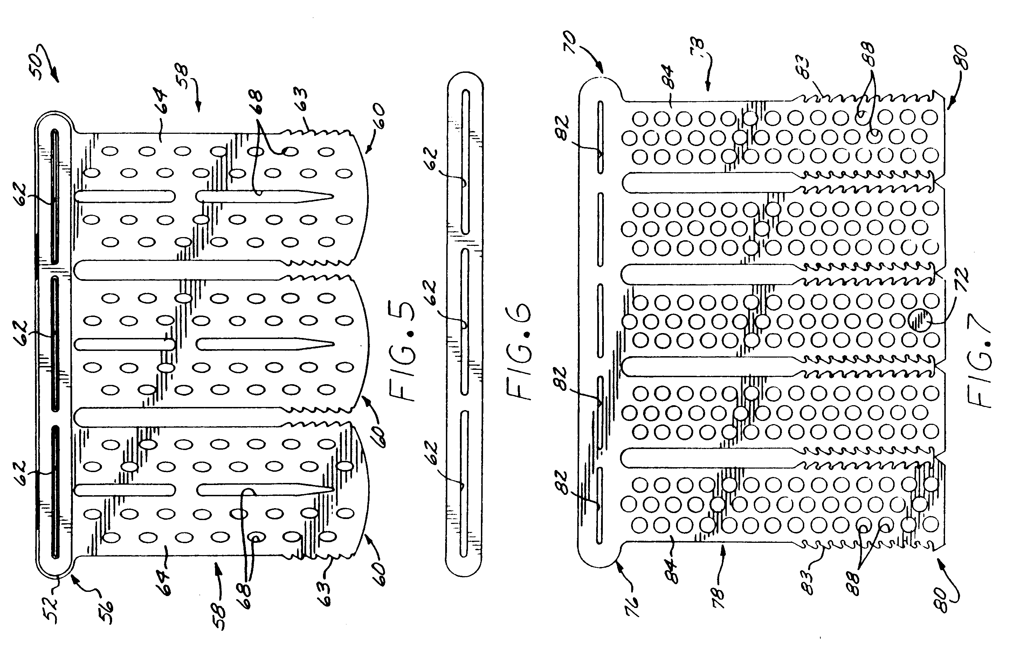

With reference to Figs. 5 and 6, in a third embodiment, the metal and polymer composite locking stent 50 comprises an elongated, planar metal structural member 52 extending across the width of the proximal first or head end 56 of the composite locking stent. The composite locking stent also comprises a plurality of main body portions 58 formed of the polymeric layers, connected to the head end and extending distally from the head end. The third embodiment of the composite locking stent currently typically also has three main body portions, although the composite locking stent can have as few as one main body portion, or more than three. Each of the main body portions includes a distal second end or tail end portion 60 opposing the head end.

The head end preferably includes a number of transverse, elongated slots 62 corresponding to the number and width of the main body portions of the composite locking stent. As in the first and second embodiments, the composite locking stent is formed so as to be capable of being rolled up into a cylindrical configuration whereby the first end overlaps the second end, and so that the tail end portions of the composite locking stent can be inserted through the corresponding slots in the head end. The metal structural member preferably is laminated on the top and bottom surfaces with a layer of a polymer 64 capable of being loaded with and releasing therapeutic drugs. In the third embodiment, the sides of the tail end portions of the polymeric layers forming the main body portions of the composite locking stent also are formed as a plurality of scalloped or lobed teeth 63 generally directed outward and distally from the head end.

The polymeric main body portions also preferably include a plurality of apertures 68 to facilitate the process of degradation and absorption of the stent once it is implanted, to allow blood flow through the stent to side branch vessels, for blood flow to the vessel wall, endothelialization, and flexibility.

The polymeric materials with which the composite locking stent are laminated in the third embodiment of the composite locking stent will be described further below. The layers of biodegradable polymeric film on either side of the metal structural member preferably are selected for the ability of the layers to be loaded with and release drugs at predictable rates when the stent is implanted in a blood vessel or other lumen in the body. The biodegradable polymeric film layers can contain the same or different drugs, or combinations of drugs, which are described further below.

In a fourth embodiment of the composite locking stent, illustrated in Fig. 7, the metal and polymer composite locking stent 70 comprises a planar sheet of the two polymeric layers 84 forming the proximal first or head end 76 of the composite locking stent, and the plurality of main body portions 78, connected to the head end and extending distally from the head end. The fourth embodiment of the composite locking stent currently typically has five main body portions, although the composite locking stent can have as few as one main body portion, or more than five. Each of the main body portions includes a distal second end or tail end portion 80 opposing the head end. The head end preferably includes a number of transverse, elongated slots 82 corresponding to the number and width of the main body portions of the composite locking stent.

The metal structural member comprises one or more planar radio-opaque markers 72 sandwiched between the two polymeric layers, typically at a tail end of a central main body portion for finding a central locus of the composite locking stent, although the markers can be placed at any suitable location in the stent. The radio-opaque marker currently preferably is made of platinum-iridum alloy, although other metals may also be suitable, as discussed below. The composite locking stent is formed so as to be capable of being rolled up into a cylindrical configuration whereby the first end overlaps the second end, and so that the tail end portions of the composite locking stent can be inserted through the corresponding slots in the head end.

The polymer layers 84 laminated on the top and bottom surfaces of the metal structural member preferably is capable of being loaded with and releasing therapeutic drugs. In the fourth embodiment, the sides of the tail end portions of the polymeric layers forming the main body portions of the composite locking stent are formed as a plurality of pronged, projecting teeth 83 generally directed outward and distally from the head end.

The polymeric main body portions also preferably include a plurality of apertures 88 to facilitate the process of degradation and absorption of the stent once it is implanted, to allow blood flow through the stent to side branch vessels, for blood flow to the vessel wall, endothelialization, and flexibility. The tail end thus is insertable through the slot so as to form a cylindrical, loop shaped stent that can be rolled and contracted for placement within a blood vessel.

The polymeric materials with which the composite locking stent are laminated in the fourth embodiment of the composite locking stent will be described further below. The layers of biodegradable polymeric film on either side of the metal structural member preferably are selected for their ability to be loaded with and release drugs at predictable rates when the stent is implanted in a blood vessel or other lumen in the body. The biodegradable polymeric film layers can contain the same or different drugs, or combinations of drugs, which are described further below.

In the first three embodiments of the composite locking stent, the metal structural member of the locking stent currently preferably is formed from a sheet of 316L stainless steel that is approximately 0.025 millimeters (0.001 inch) thick. Stainless steel is advantageous because of its low carbon content and because it contains molybdenum, which greatly increases corrosion resistance. For each of the embodiments, the metal structural member can be selected from metals such as stainless steel, tantalum, nickel-titanium alloy, platinum-iridium alloy, molybdenum-rhenium alloy, gold, magnesium, combinations thereof, although other similar materials also may be suitable. The metal can be modified to exhibit different hardnesses, and thus varying stiffnesses, by well known annealing and manufacturing processes. The sheet of thin metal can be cut in the desired shape to form the metal structural member with a laser, such as a continuous CO₂ laser, a pulsed YAG laser, or an excimer laser, for example, or alternatively, by chemical etching or stamping.

The polymeric materials with which the metal structural member of the composite locking stent of the invention is laminated preferably comprise a biodegradable, bioabsorbable polymeric film that is capable of being loaded with and releasing therapeutic drugs, preferably include, but are not limited to, polycaprolactone (PCL), poly-DL-lactic acid (DL-PLA) and poly-L-lactic acid (L-PLA) or lactide. Other biodegradable, bioabsorbable polymers such as polyorthoesters, polyiminocarbonates, aliphatic polycarbonates, and polyphosphazenes also may be suitable, and other non-degradable polymers capable of carrying and delivering therapeutic drugs also may be suitable. At least one of the polymeric layers is to be loaded with a pharmacologic agent for use in localized drug therapy. As used in this description, the terms biodegradable, bioabsorbable, reabsorbable, degradable, and absorbable are meant to encompass materials that are broken down and gradually absorbed or eliminated by the body, whether these processes are due to hydrolysis, metabolic processes, bulk or surface erosion. The primary purpose of the polymeric layers is to carry one or more therapeutic drugs, and to provide a launch platform for localized delivery of the therapeutic drugs. In each of the foregoing embodiments, one polymeric layer typically currently preferably is about 2.54 millimeters (0.002 inch) thick poly (L) lactide, and the other polymeric layer is typically preferably about 0.025 millimeters (0.001 inch) thick polycaprolactone, both of which are bioabsorbable.

The thin polymeric films used in forming the stent are preferably first intermixed with the drug or drugs to be delivered, and then typically are laminated or solvent cast to the surface of the metal structural member. Lamination processing methods and temperatures can vary widely depending on the polymers used and the temperature sensitivity of the loaded drugs. Alternatively, the metal structure of the stent can be encapsulated in the layers of polymeric material by solvent casting, melt processing, insert molding, and dip coating. Apertures in the polymeric material can be cut with a laser, such as a continuous CO₂ laser, a pulsed YAG laser, or an excimer laser, for example, or alternatively, by chemical etching or stamping.

In preferred embodiments of the invention, a therapeutic drug or agent is combined with at least one of the two polymeric layers laminating the metal structural member of the stent, for purposes of diffusing the drug into the vessel of the patient. Examples of therapeutic drugs, or agents that can be combined with the polymeric layers include antiplatelets, antithrombins, and antiproliferatives. Examples of antiplatelets and antithrombins include but are not limited to sodium heparin, low molecular weight heparin, hirudin, argatroban, forskolin, vapiprost, prostacyclin and prostacyclin analogues, dextran, D-phe-pro-arg-chloromethylketone (synthetic antithrombin), dipyridamole, glycoprotein IIb/IIIa platelet membrane receptor antibody, recombinant hirudin, thrombin inhibitor (available from Biogen), and 7E-3B (an antiplatelet drug from Centocore). Examples of cytostatic or antiproliferative agents include angiopeptin (a somatostatin analogue from Ibsen), angiotensin converting enzyme inhibitors such as Captopril (available from Squibb), Cilazapril (available from Hoffman-LaRoche), or Lisinopril (available from the Merck Co.); calcium channel blockers (such as Nifedipine), colchicine, fibroblast growth factor (FGF) antagonists, fish oil (omega 3-fatty acid), histamine antagonists, Lovastatin (an inhibitor of HMG-CoA reductase, a cholesterol lowering drug from the Merck Co.), methotrexate, monoclonal antibodies (such as to PDGF receptors), nitroprusside, phosphodiesterase inhibitors, prostaglandin inhibitor (available from Glaxo), Seramin (a PDGF antagonist), serotonin blockers, steroids, thioprotease inhibitors, triazolopyrimidine (a PDGF antagonist), and nitric oxide. Other therapeutic drugs or agents which may be appropriate include alpha-interferon and genetically engineered epithelial cells, for example. While the foregoing therapeutic agents have been used to prevent or treat restenosis, they are provided by way of example and are not meant to be limiting, since other therapeutic drugs may be developed which are equally applicable for use with the present invention.

In each of the foregoing embodiments, the tail end thus is insertable through the slot so as to form a cylindrically, loop shaped stent that can be rolled and contracted for placement within a blood vessel. The stent can be placed in a blood vessel in a rolled, cylindrical, contracted loop configuration with a sufficiently small outer diameter so as to be transportable through the targeted blood vessel or other lumen, and of a sufficiently large internal diameter to receive an inflation balloon device (not shown) therein. The stent thus can be urged into an unfurled, expanded configuration by inflation of the inflation balloon device, and locked in the desired expanded configuration by the locking of the teeth on the periphery of the tail portion in the corresponding slot of the head end, so that the stent cannot recontract.

It thus has been demonstrated that the described embodiments provide for a composite locking stent that provides the structure and mechanical strength required for a stent, and that provides the capability of being loaded with therapeutic drugs, to function as a stent for delivery and release of the therapeutic drugs at a specific intravascular site.

It therefore will be apparent from the foregoing that while particular forms of the invention have been illustrated and described, various modifications can be made without departing from the scope of the invention. Accordingly, it is not intended that the invention be limited, except as by the appended claims.

## Claims

1. A composite locking stent, comprising:
a proximal head end portion;
at least one elongated main body portion connected to said head end portion, said at least one main body portion extending distally from said head end portion, each said main body portion having a tail end portion opposing said head end portion;
at least one elongated slot disposed in said head end portion extending transversely to said elongated main body portion, each said tail end portion being adapted to interlock with said head end portion through a corresponding one of said slots in said head end portion; and
metal structural means disposed in at least a portion of one of said head end portion and said at least one main body portion for providing structural support to said composite locking stent, said metal structural means being disposed between first and second layers of polymeric material laminated together, said first and second layers of polymeric material forming an exterior portion of said head end portion and said at least one main body portion, and at least one of said layers of polymeric material being capable of being loaded with and releasing therapeutic agents at a predictable rate for delivery of the therapeutic agents in localized drug therapy in a blood vessel.

2. The composite locking stent of Claim 1, wherein said metal structural means comprises a planar metal structural member providing the structural support in said head end portion.

3. The composite locking stent of Claim 1, wherein said metal structural means comprises a planar metal structural member providing the structural support in said head end portion and in said at least one main body portion.

4. The composite locking stent of Claim 3, wherein each said planar metal structural member disposed in said tail end portion of said at least one main body portion comprises peripheral edges having a plurality of teeth directed outward from said tail end portion and distally from said head end portion for engaging said corresponding slot in said head end portion.

5. The composite locking stent of Claim 1, wherein said metal structural member in said at least one main body portion has a surface defining at least one aperture.

6. A composite stent, comprising:
a first layer of polymeric material;
a second layer of a planar metal structural member providing structural support for said composite stent, said second layer having a first side and a second side, and said first layer of polymeric material being laminated to said first side of said second layer; and
a third layer of polymeric material laminated to said second side of said second layer, at least one of said layers of polymeric material being loaded with at least one therapeutic agent to be delivered at a predictable rate in localized drug therapy in a blood vessel.

7. The composite stent of Claim 6, wherein at least one of said layers defines a proximal head end portion and at least one elongated main body portion connected to said head end portion, said at least one main body portion extending distally from said head end portion, each said elongated main body portion having a tail end portion opposing said head end portion.

8. The composite stent of Claim 7, wherein said second layer of said planar metal structural member comprises provides structural support in said head end portion and in said at least one main body portion.

9. The composite stent of Claim 1 or Claim 6, wherein said metal structural means comprises a radio-opaque marker.

10. The composite stent of Claim 1 or Claim 6, wherein at least one of said first and second layers of polymeric material is selected from the group consisting of polycaprolactone, poly-DL-lactic acid, poly-L-lactic acid, polyorthoesters, polyiminocarbonates, aliphatic polycarbonates, and polyphosphazenes.

11. The composite stent of Claim 1 or Claim 6, wherein at least one of said layers of polymeric material contains a therapeutic agent selected from the group consisting of antiplatelets, antithrombins, cytostatic and antiproliferative agents, and combinations thereof.

12. The composite stent of Claim 1 or Claim 6, wherein at least one of said layers of polymeric material contains a therapeutic agent selected from the group consisting of sodium heparin, low molecular weight heparin, hirudin, argatroban, forskolin, vapiprost, prostacyclin and prostacyclin analogues, dextran, D-phe-pro-arg-chloromethylketone, dipyridamole, glycoprotein IIb/IIIa platelet membrane receptor antibody, recombinant hirudin, thrombin inhibitor, angiopeptin, angiotensin converting enzyme inhibitors, or Lisinopril; calcium channel blockers, colchicine, fibroblast growth factor antagonists, fish oil, omega 3-fatty acid, histamine antagonists, HMG-CoA reductase inhibitor, methotrexate, monoclonal antibodies, nitroprusside, phosphodiesterase inhibitors, prostaglandin inhibitor, serotonin blockers, steroids, thioprotease inhibitors, triazolopyrimidine and other PDGF antagonists, alpha-interferon, genetically engineered epithelial cells, nitric oxide, and combinations thereof.

13. The composite stent of Claim 7, wherein said second layer of said metal structural member is disposed in said at least one main body portion and has a surface defining at least one aperture.

14. The composite stent of Claim 5 or Claim 13, wherein said at least one aperture is filled in by said layers of polymeric material, and said layers of polymeric material have a surface defining a plurality of apertures through said polymeric material and said at least one aperture.

15. The composite stent of Claim 1 or Claim 6, wherein said layers of polymeric material are bioabsorbable.

16. The composite stent of Claim 7, wherein at least one elongated slot is disposed in said head end portion extending transversely to said elongated main body portion, each said tail end portion being adapted to interlock with said head end portion through a corresponding one of said slots in said head end portion.

17. The composite stent of Claim 1 or Claim 16, wherein each said tail end portion of said at least one main body portion comprises peripheral edges having a plurality of teeth directed outward from said tail end portion and distally from said head end portion for engaging said corresponding slot in said head end portion.

18. The composite stent of Claim 17 when dependent upon Claim 16, wherein said second layer of said planar metal structural member comprises is disposed in said tail end portion of said at least one main body portion having peripheral edges with a plurality of teeth directed outward from said tail end portion and distally from said head end portion for engaging said corresponding slot in said head end portion.

19. The composite stent of Claim 17 when dependent upon Claim 16, wherein said plurality of teeth are formed by said first and third layers of polymeric material.

20. The composite locking stent of Claim 16 when dependent upon Claim 1, wherein said plurality of teeth are formed by said first and second layers of polymeric material.
